# DEMANDE DE BREVET EUROPEEN

(11) **EP 0 556 893 A1**
(43) Date de publication de la demande: **25.08.1993**
(21) Numéro de dépôt: 93200321.3
(22) Date de dépôt: 05.02.1993
(51) Int. Cl.: C07C 17/00, C07C 21/18

(54) **Procédé pour la préparation de 1,1-difluoro-2-chloroéthylène au départ de 1,1-difluoro-1,2,2-trichloroéthane**

(30) Priorité: 17.02.1992 BE 9200164
(71) Demandeur: SOLVAY (Société Anonyme), B-1050 Bruxelles (BE)
(72) Inventeur: Vanlautem, Noel, B-1300 Wavre (BE); Wilmet, Vincent, B-1301 Wavre (BE); Pirotton, Joseph, B-1030 Bruxelles (BE); Lerot, Luc, B-1200 Bruxelles (BE)
(74) Mandataire: Marckx, Frieda

(57) **Abrégé**

Procédé pour la préparation de 1,1-difluoro-2-chloroéthylène par réaction en phase gazeuse de 1,1-difluoro-1,2,2-trichloroéthane en présence d'hydrogène et d'une composition catalytique comprenant un support à base de carbone sur lequel sont déposés du cuivre et au moins un métal du groupe VIII du tableau périodique des éléments.

## Description

La présente invention concerne un procédé pour la préparation de 1,1-difluoro-2-chloroéthylène au départ de 1,1-difluoro-1,2,2-trichloroéthane.

Le 1,1-difluoro-2-chloroéthylène est un monomère intéressant pour la fabrication de polymères, homo- et copolymères chlorofluorés et comme intermédiaire pour la préparation d'autres composés fluorés et chlorofluorés.

Dans la demande de brevet européen EP-A-0 253 410 au nom de AUSIMONT, on décrit la préparation de fluoroéthylènes et de chlorofluoroéthylènes par réaction en phase gazeuse de chlorofluoroéthanes en présence d'hydrogène à l'intervention de catalyseurs supportés sur carbone constitués de palladium, de nickel, de chrome, de cobalt, de platine, de cuivre ou de leurs mélanges, la préférence étant donnée au palladium et au nickel. Toutefois, l'application de ce procédé au départ de 1,1-difluoro-1,2,2-trichloroéthane n'est pas mentionnée. Le 1,1-difluoro-2-chloroéthylène est par contre obtenu au départ de difluorotétrachloroéthane.

La présente invention a pour but de fournir un procédé pour la préparation de 1,1-difluoro-2-chloroéthylène avec un taux de transformation de 1,1-difluoro-1,2,2-trichloroéthane et une sélectivité en 1,1-difluoro-2-chloroéthylène particulièrement intéressants.

A cet effet, l'invention concerne un procédé pour la préparation de 1,1-difluoro-2-chloroéthylène par réaction en phase gazeuse de 1,1-difluoro-1,2,2-trichloroéthane en présence d'hydrogène et d'une composition catalytique comprenant un support à base de carbone sur lequel sont déposés du cuivre et au moins un métal du groupe VIII du tableau périodique des éléments.

Le procédé selon l'invention offre d'excellents résultats se traduisant notamment par un taux de transformation de 1,1-difluoro-1,2,2-trichloroéthane très élevé et une sélectivité élevée en 1,1-difluoro-2-chloroéthylène. Ce procédé est également aisé à mettre en oeuvre et offre une bonne stabilité de la composition catalytique.

Parmi les métaux du groupe VIII du tableau périodique des éléments, utilisables seuls ou en mélange, on donne la préférence au ruthénium, au rhodium, à l'iridium, au platine et au palladium et plus particulièrement encore à l'iridium, au platine et au palladium. Les métaux du groupe VIII du tableau périodique des éléments tout particulièrement préférés sont le platine et le palladium. Les meilleurs résultats sont obtenus avec le palladium.

Le procédé selon l'invention met en oeuvre une composition catalytique qui comprend généralement au moins 0,05 % en poids de métal du groupe VIII du tableau périodique des éléments, par rapport au poids total de la composition catalytique. De préférence, elle en comprend au moins 1 %. Par ailleurs, la composition catalytique mise en oeuvre ne comprend habituellement pas plus de 10 % en poids de métal du groupe VIII du tableau périodique des éléments, par rapport au poids total de la composition catalytique. De préférence, elle n'en comprend pas plus de 5 %.

Le procédé selon l'invention met en oeuvre une composition catalytique qui comprend généralement au moins 0,1 % en poids de cuivre par rapport au poids total de la composition catalytique. De préférence, elle comprend au moins 1 % en poids de cuivre. Par ailleurs, on n'excède habituellement pas 50 % en poids de cuivre, par rapport au poids total de la composition catalytique. De préférence, on ne dépasse pas 30 %.

Le rapport en poids entre le cuivre et le métal du groupe VIII du tableau périodique des éléments dans la composition catalytique peut varier dans de larges limites. Le plus souvent, il est d'au moins 0,1 sans excéder 20. On travaille avantageusement avec un rapport d'au moins 0,4. De préférence, on ne dépasse pas un rapport de 10.

Le cuivre et le métal (ou les métaux) du groupe VIII du tableau périodique des éléments sont généralement mis en oeuvre dans le procédé selon l'invention sous forme de composés organiques ou inorganiques. Comme composés organiques utilisables, on peut mentionner les carboxylates, les alcoolates et les acétylacétonates qui contiennent de 1 à 10 atomes de carbone. Comme composés inorganiques utilisables, on peut mentionner les halogénures, les hydroxydes et les nitrates et plus particulièrement, les halogénures et les hydroxydes. De manière avantageuse, on choisit les chlorures, fluorures et hydroxydes. On obtient d'excellents résultats avec les chlorures.

Le support à base de carbone est généralement constitué de charbon actif ayant un important volume poreux. Le plus souvent, ce volume poreux est compris entre 0,1 et 2 cm³/g. Il est de préférence compris entre 0,2 et 1 cm³/g.

La surface spécifique du support à base de carbone est généralement comprise entre 10 et 1.500 m²/g.

Le procédé selon l'invention met en oeuvre une composition catalytique qui peut être obtenue par imprégnation du support avec les solutions de composés métalliques. L'imprégnation du support peut être réalisée par n'importe quelle méthode. En pratique, elle peut être obtenue par la technique dite du "volume poreux" (imprégnation dite "sèche") ou par la technique du "volume excédentaire" (imprégnation dite "humide"). De telles méthodes sont décrites dans la littérature, en particulier par Charles N. Satterfield "Heterogeneous catalysis in practice", 1980, Mc Graw-Hill, New-York, spécialement p. 82 et 83, qui est incorporé à la présente demande par référence.

Les solutions d'imprégnation peuvent être aqueuses ou organiques. De préférence, on met en oeuvre une solution aqueuse ou alcoolique.

L'ordre d'imprégnation du support n'est pas critique. L'imprégnation peut donc être réalisée d'abord avec une solution comprenant le cuivre ou encore d'abord avec une solution comprenant au moins un métal du groupe VIII du tableau périodique des éléments, ou simultanément avec ces deux solutions, ou avec une solution contenant le cuivre et au moins un métal du groupe VIII.

Après imprégnation, le support peut être séché avant d'être introduit dans le réacteur proprement dit.

La composition catalytique ainsi obtenue peut être mise en oeuvre dans le procédé selon l'invention telle quelle ou peut être préalablement réduite soit par de l'hydrogène, soit par un mélange d'hydrogène avec un gaz inerte tel que par exemple l'azote ou l'hélium.

La température à laquelle s'effectue la réaction selon le procédé de l'invention est généralement d'au moins 80 °C. De préférence, cette température est d'au moins 120 °C et plus particulièrement encore d'au moins 200 °C. Par ailleurs, il est préférable qu'elle n'excède pas 400 °C et plus particulièrement qu'elle ne dépasse pas 300 °C.

La pression sous laquelle est effectuée la réaction n'est pas critique en elle-même. Habituellement, on opère sous une pression comprise entre 1x10⁵ et 10x10⁵ pascals. De préférence, on retient une pression comprise entre 2x10⁵ et 5x10⁵ pascals.

Le rapport molaire entre l'hydrogène et le 1,1-difluoro-1,2,2-trichloroéthane mis en oeuvre est généralement d'au moins 0,25. De préférence, il est d'au moins 0,4 et plus particulièrement encore d'au moins 1. Par ailleurs, ce rapport molaire ne dépasse habituellement pas 20. De préférence, il n'excède pas 10. Avec un rapport situé aux environs de 4 à 9, d'excellents résultats ont été obtenus qui se traduisent en particulier par une très grande stabilité de la composition catalytique.

Le temps de contact moyen entre la composition catalytique et les produits en réaction est généralement d'au moins 0,5 seconde. De préférence, il est d'au moins 1 seconde. Par ailleurs, ce temps de contact ne dépasse habituellement pas 10 secondes. De préférence, il ne dépasse pas 6 secondes.

Le procédé selon l'invention peut être réalisé en présence d'un gaz inerte tel que par exemple l'azote ou l'hélium.

Le procédé selon l'invention peut être mis en oeuvre dans tous types de réacteurs. En particulier, il peut aussi bien être mis en oeuvre dans un réacteur à lit fixe que dans un réacteur à lit fluidisé. D'excellents résultats ont été obtenus dans un réacteur à lit fixe. Lorsque l'on met en oeuvre le procédé selon l'invention dans un réacteur à lit fluidisé, il peut être nécessaire d'adapter à cette technique par ailleurs connue les conditions opératoires ci-dessus décrites, notamment en ce qui concerne la pression, la température et en particulier le temps de séjour.

Les exemples non limitatifs qui suivent sont donnés dans le but d'illustrer l'invention.

### Exemple 1

### a) Préparation de la composition catalytique

Dans une ampoule de 50 cm³, on introduit 20 cm³, soit 7 g d'un support à base de carbone (C LURGI ASIV 420), d'une surface spécifique de 1.100 m²/g et d'un volume poreux de 0,6 cm³/g.

On chauffe l'ampoule sous vide (1 à 600 Pa) à 125 °C pendant 2 heures en vue de sécher et de dégazer le support.

Après refroidissement, le support est imprégné sous vide à température ambiante par 4 cm³ d'une solution aqueuse comprenant 1,75 g de chlorure cuivrique. Ensuite, le support est imprégné avec 4 cm³ d'une solution aqueuse à 10 % en volume d'acide chlorhydrique concentré comprenant 0,33 g de chlorure de palladium. On laisse l'imprégnation se poursuivre pendant 2 heures sous vide et environ 16 heures à pression atmosphérique à température ambiante.

On sèche ensuite durant 3 heures à la pression atmosphérique à 120 °C.

La composition catalytique ainsi obtenue comprend 9,1 % en poids de cuivre et 2,2 % en poids de palladium par rapport au poids total de la composition catalytique.

1 cm³ de cette composition catalytique est introduit dans un réacteur constitué d'un tube métallique en inox long de 520 mm et d'un diamètre intérieur de 7,7 mm ; la composition catalytique est ensuite conditionnée 2 heures à 240 °C sous 3x10⁵ pascals au moyen d'un mélange d'hydrogène et d'hélium dans un rapport volumique 10/90 à un débit de 40 cm³/min.

### b) Hydrogénation du 1,1-difluoro-1,2,2-trichloroéthane

Le réacteur est alimenté à raison de 0,0054 mole/heure de 1,1-difluoro-1,2,2-trichloroéthane et de 0,0486 mole/heure d'hydrogène à 240 °C sous 3x10⁵ pascals. Le temps de séjour moyen est évalué à 4,7 s.

Après 10 heures de fonctionnement, le taux de transformation du 1,1-difluoro-1,2,2-trichloroéthane est de 100 %, et la sélectivité en 1,1-difluoro-2-chloroéthylène est de 88 %, en mole.

### Exemple 2

La composition catalytique utilisée est celle décrite à l'exemple 1.

1 cm³ de cette composition catalytique est conditionné comme dans l'exemple 1, dans un réacteur identique.

Le réacteur est alimenté à raison de 0,0108 mole/heure de 1,1-difluoro-1,2,2-trichloroéthane, de 0,0486 mole/heure d'hydrogène, et de 0,0486 mole/heure d'hélium à 240 °C sous 3x10⁵ pascals. Le temps de séjour moyen est évalué à 2,4 s.

Après 10 heures de fonctionnement, le taux de transformation du 1,1-difluoro-1,2,2-trichloroéthane est de 100 %, la sélectivité en 1,1-difluoro-2-chloroéthylène est de 92 % en mole.

## Revendications

1. Procédé pour la préparation de 1,1-difluoro-2-chloroéthylène par réaction en phase gazeuse de 1,1-difluoro-1,2,2-trichloroéthane en présence d'hydrogène et d'une composition catalytique, caractérisé en ce que la composition catalytique comprend un support à base de carbone sur lequel sont déposés du cuivre et au moins un métal du groupe VIII du tableau périodique des éléments.

2. Procédé selon la revendication 1, caractérisé en ce que le métal du groupe VIII du tableau périodique des éléments est choisi parmi le ruthénium, le rhodium, l'iridium, le platine, le palladium et leurs mélanges.

3. Procédé selon la revendication 2, caractérisé en ce que le métal du groupe VIII du tableau périodique des éléments est choisi parmi l'iridium, le platine, le palladium et leurs mélanges.

4. Procédé selon la revendication 3, caractérisé en ce que le métal du groupe VIII du tableau périodique des éléments est choisi parmi le platine, le palladium et leurs mélanges.

5. Procédé selon la revendication 4, caractérisé en ce que le métal du groupe VIII du tableau périodique des éléments est le palladium.

6. Procédé selon la revendication 1, caractérisé en ce que la composition catalytique comprend de 0,05 à 10 % en poids de métal du groupe VIII du tableau périodique des éléments par rapport au poids total de la composition catalytique.

7. Procédé selon la revendication 6, caractérisé en ce que la composition catalytique comprend de 1 à 5 % en poids de métal du groupe VIII du tableau périodique des éléments par rapport au poids total de la composition catalytique.

8. Procédé selon la revendication 1, caractérisé en ce que la composition catalytique comprend de 0,1 à 50 % en poids de cuivre par rapport au poids total de la composition catalytique.

9. Procédé selon la revendication 8, caractérisé en ce que la composition catalytique comprend de 1 à 30 % en poids de cuivre par rapport au poids total de la composition catalytique.

10. Procédé selon la revendication 1, caractérisé en ce que le rapport volumique entre l'hydrogène et le 1,1-difluoro-1,2,2-trichloroéthane mis en oeuvre est d'au moins 1 et ne dépasse pas 10.
